# EUROPÄISCHE PATENTANMELDUNG

(11) **EP 1 978 086 A1**
(43) Veröffentlichungstag der Anmeldung: **08.10.2008**
(21) Anmeldenummer: 08004630.3
(22) Anmeldetag: 12.03.2008
(51) Int. Cl.: C12M 1/107, C12P 5/02, C12P 7/10

(54) **Verfahren zum Hybridaufschluss von lignocellulosehaltiger Biomasse**

(30) Priorität: 13.03.2007 DE 102007012104
(71) Anmelder: Stirl Anlagentechnik GmbH, 18292 Krakow a.S. (DE)
(72) Erfinder: Stirl, Armin, Dr., 18292 Krakow am See (DE); Gerath, Horst, Prof., Dr., 23999 Malchow/Insel Poel (DE); Knitter, Christian, Dipl.-Ing., 18057 Rostock (DE)
(74) Vertreter: Peters, Hajo

(57) **Zusammenfassung**

Die vorliegende Erfindung betrifft ein Verfahren zum Hybridaufschluss von lignocellulosehaltiger Biomasse durch Zerfaserung und Hydrolyse mit der Gerätekombination bekannter Zerfaserungsgeräte und einer Hochdruckmikrowelle - zur Nutzung in Biogas- und Bioethanol-Produktionsprozessen, sowie Vefahrensvorrichtungen geeignet zur Durchführung des erfindungsgemäßen Verfahrens.

## Beschreibung

Die vorliegende Erfindung betrifft ein Verfahren zum Hybridaufschluss von wasser- und lignocellulosehaltiger Biomasse durch Zerfaserung und Hydrolyse mit der Gerätekombination bekannter Zerfaserungsgeräte und einer Hochdruckmikrowelle - zur Nutzung in Biogas- und Bioethanol-Produktionsprozessen, sowie Vefahrensvorrichtungen geeignet zur Durchführung des erfindungsgemäßen Verfahrens.

In den letzten Jahrzehnten ist weltweit der Bedarf an Formen der Energiegewinnung gestiegen, die eine Alternative zu Kernkraft und der Verwendung fossiler Brennstoffe bilden. Eine dieser Alternativen ist die Gewinnung von Biogas oder auch Bioethanol aus wasser- und lignocellulosehaltiger Biomasse (feuchter Biomasse). Als Rohstoff können beispielsweise Energegräser, Silagen aus Mais, Gras- oder Getreidepflanzen, aber auch Stroh oder Dung dienen. Dieses Biogas wird dann in Strom umgewandelt oder zur Wärmegewinnung eingesetzt. Gerade diese Nutzung nachwachsender Rohstoffe bzw. von Nebenprodukten aus der Landwirtschaft bietet damit interessante wirtschaftliche Möglichkeiten für den ländlichen Raum. Darüber hinaus hat die Erzeugung erneuerbarer Energien (Biogas und Bioethanol) klimarelevante Bedeutung.

Es sind im Laufe der letzten Jahre verschiedene Vorschläge zur Verbesserung der Biogasproduktion vorgeschlagen worden. Eines dieser Beispiele ist die DE 10 2004 054 468 A1 in der die Biomasse vor dem Vergärungsprozeß durch einen Extruder homogenisiert und zerkleinert wird. Allerdings ist auch bei diesem Verfahren erhebliches Verbesserungspotential in der Ausnutzung der in der Biomasse verfügbaren Energie erkennbar.

Der Erfindung liegt daher die Aufgabe zugrunde, ein Verfahren zur Verfügung zu stellen, das erlaubt, das in der Biomasse vorhandene energetische Potential besser zu nutzen. Dazu gehört neben der Erhöhung der Biogasausbeute natürlich auch die Verkürzung der bei Biogas üblichen Verweil- bzw. Vergärungszeiten im Faulraum eines Biogasreaktors. Ebenso wichtig ist auf der anderen Seite die Erhöhung der Zuckerausbeuten aus wasser- und lignocellulosehaltiger Biomasse für die Vergärung zu größeren Bioethanolmengen, ohne dass sich zustzliche Inhibitoren (Zuckerverfallsprodukte) bilden.

Erfindungsgemäß wird die Aufgabe gelöst durch ein Verfahrenzur Herstellungvon Biogas oder Bioethanol aus wasser- und lignocellulosehaltiger Biomasse, in dem
• in einer Stufe (a) lignocellulosehaltige Biomasse durch ein Zerfaserungsgerät aufbereitet wird;
• in einer nachfolgenden Stufe (b) aufbereitete lignocellulosehaltige Biomasse aus Stufe (a) in einer thermischen Hydrolyse (b1) in einer Hochdruckmikrowelle erwärmt und hydrolysiert wird; und
• in einer nachfolgenden Stufe (c) hydrolysierte lignocellulosehaltige Biomasse aus Stufe (b) einem anaeroben Vergärungsprozess unterworfen wird.

Dabei ist "Biomasse" definiert als eine Masse überwiegend organischer Materialien pflanzlicher Herkunft. "Lignocellulose" ist definiert als Kombination aus Lignin, Hemicellulosen und Cellulose, die das Strukturgerüst der pflanzlichen Zellwand bildet. Dementsprechend ist "lignocellulosehaltige Biomasse" definiert als eine Masse organischer Materialien pflanzlicher Herkunft mit pflanzlicher Zellwandstruktur und umfasst beispielsweise Mais, Maissilage, Gras, Grassilage, Ganzpflanzengetreide, Silage aus Getreideganzpflanzen, Stroh, Dung, aber auch Garten- oder Forstabfall wie Zweige und andere Grünschnitte.

Die "Aufbereitung" der lignocellulosehaltigen Biomasse "durch ein Zerfaserungsgerät" ist definiert als mechanischer Aufschluß der Biomasse, in der diese so behandelt, vorzugsweise zerfasert wird, dass die morphologische Makrostatur der Biomasse ganz oder teilweise zerstört bzw. beschädigt wird. Dabei wird der Zellsaft teilweise ausgepresst und die feste Biomasse zerfasert. Diese Aufbereitung findet bevorzugt als Extrusion mit einem Extruder statt, und erfolgt beispielsweise mit einem als Einfach- oder Doppelschneckenextruder bzw. Doppelkammerextruder.

Die "thermische Hydrolyse" der Biomasse "in einer Hochdruckmikrowelle" wird im Rahmen dieser Erfindung so definiert, dass die wasserhaltige Biomasse in kurzer Zeit auf weit über 100°C erhitzt wird, so dass durch die hohen Temperaturen und das verdampfte Wasser aus der Biomasse thermische Hydrolyse stattfindet. Die Temperaturerhöhung erfolgt dabei unter starkem Druckanstieg,vorzugsweise auf 5 bis 25 bar, bis auf beispielsweie auf 160 bis 220° C.

Der "anaerobe Vergärungsprozess", dem die Biomasse unterworfen wird, ist ein Prozess, in dem die Biomasse in einem Reaktor, beispielsweise einem Faulraum/Faulbehälter/Fermenter, unter Luftabschluß einem Stoffwechselprozess unterworfen wird, wobei vorzugsweise Enzyme oder Mikroorganismen zum Einsatz kommen.

Das erfindungsgemäße Verfahren wie auch die erfindungsgemäßen Verfahrensvorrichtungen, in denen dieses Verfahren durchgeführt werden kann, zeigen eine Reihe von Vorteilen gegenüber den bisher angebotenen Lösungen. So führt das Verfahren im Biogasprozess zu einer deutlichen Verkürzung der Verweilzeit im Biogasreaktor und zu höheren Biogasausbeuten, was auf eine verkürzte und vollständigere Hydrolyse (acidogene Phase) im Reaktor zurückzuführen ist. Entsprechend kann entweder das Reaktorvolumen der Biogasanlage reduziert oder der Rohstoffdurchsatz erhöht und somit die Biogasproduktion deutlich gesteigert werden.

Im Bioethanolprozess führt das erfindungsgemäße Verfahren durch die nahezu schlagartige Erhitzung der Biomasse zu einer sehr schnellen thermischen Hydrolyse mit hoher Verzuckerung und geringem Zuckerzerfall. Entsprechend steht mehr Zucker für die Vergärung zur Verfügung und es bilden sich weniger Inhibitoren (Furfural und 5-Hydroxymethylfurfural). Dementsprechend problemlos verläuft die Vergärung zu Ethanol und die Ausbeute an Ethanol steigt. Außerdem erlaubt das vorliegende Verfahren wie auch die beanspruchte Anlage den Biogas- bzw- Bioethanol-Herstellungsprozeß den kontinuierlichen Betrieb und beispielsweise den Verzicht auf Maischebehälter.

Die Zerfaserung mit dem Zerfaserungsgerät in Schritt (a) erlaubt es, die morphologische Makrostatur der Biomasse zu zerstören bzw. zu beschädigen und die Oberfläche damit zu vergrößern. Gleichzeitig wird die Biomasse stärker homogenosiert, was sowohl die spätere Weiterverarbeitung als auch den Weitertransport erleichtert. Außerdem setzt der mechanische Auschluß der Zerfaserung, insbesondere die Extrusion thermische Energie frei, die zu einer Erwärmung der Biomasse führt, was sich in oder in Vorbereitung auf spätere/n Verfahrensschritte(n (insbesondere Schritt (b)) günstig auswirken kann.

Die thermische Hydrolyse in Schritt (b) bewirkt eine Beschädigung bzw. Aufspaltung des Lignoceellulose-Komplexes, so dass die Cellulose und die Hemicellulose für Mikroorganismen und/oder Enzyme leichter angreifbarer werden.

Gerade die Kombination aus vorangehender Zerfaserung /Extrusion und folgender Mikrowellenbehandlung, erlaubt zum einen einen besonders effektiven Schritt (b), aber eben auch eine besonders günstige Vorbereitung des nachfolgenden Schritts (c).

In Schritt (c), der anaeroben Vergärung, wird die Bomasse aus den vorangegangenen Verfahrensschritten in Reaktoren, bzw. Fermentern enzymatisch oder mikrobiologisch umgesetzt. Die in den Schritten (a) und (b) erreichten Vorteile, die sich insbesondere in dieser Kombination gegenseitig verstärken, führen im letzten Schritt zu einer Verbesserung der Ausbeute und zu geringeren Verweilzeiten im Faulraum bzw. einer energetisch effizienteren Ausnutzung des Faulraums.

In einer bevorzugten Ausführungsform wird in diesem erfindungsgemäßen Verfahren aufbereitete lignocellulosehaltige Biomasse aus Stufe (a) vor Durchlaufen der Stufe (b) in einer Stufe (d) einem anaeroben Vorvergärungsprozess unterworfen.

In einer weiteren bevorzugten Ausführungsform wird in in einem Rezyklisierungsschritt (e) Biomasse aus dem anaeroben Vergärungsprozess der Stufe (c) erneut in einer Stufe (b) einer thermischen Hydrolyse (b1) in einer Hochdruckmikrowelle unterzogen und anschließend erneut einem anaeroben Vergärungsprozess in einer Stufe (c) unterworfen wird. Dabei kann es weiter bevorzugt sein, wenn der Rezyklisierungsschritt (e), in dem Biomasse aus einem anaeroben Vergärungsprozess der Stufe (c) erneut in einer Stufe (b) einer thermischen Hydrolyse (b1) in einer Hochdruckmikrowelle unterzogen wird und anschließend erneut einem anaeroben Vergärungsprozess in einer Stufe (c) unterworfen wird, konsekutiv mehrfach durchgeführt wird. Vorzugsweise wird vor dem Rezyklisierungsschritt (e) oder vor einem, mehreren oder allen der Rezyklisierungsschritte (e) die Biomasse aus dem anaeroben Vergärungsprozess der Stufe (c) vor Zuführung zur Stufe (b) in einer Stufe (a) durch ein Zerfaserungsgerät aufbereitet wird.

In einer weiteren bevorzugten Ausführungsform wird in diesem erfindungsgemäßen Verfahren die Zerfaserung in Stufe (a) als Extrusion mit einem Extruder ausgelegt, insbesondere erfolgt sie in Stufe (a) mit einem als Einfach- oder Doppelschneckenextruder bzw. Doppelkammerextruder ausgeführten Extruder.

In einer weiteren bevorzugten Ausführungsform wird in diesem erfindungsgemäßen Verfahren in Stufe (b)
• Biomasse in der thermischen Hydrolyse (b1) in der Hochdruckmikrowelle auf Temperaturen zwischen 150 und 250 °C erwärmt und hydrolysiert wird; und/oder
• anschließend in einem Druck-reduzierungsschritt (b2) der in der Hochdruckmikrowelle entstandene Druck in der Biomasse durch Kontaktieren der die Biomasse führenden Vorrichtung mit einem Raum mit niedrigerem Gasdruck reduziert wird.

In einer weiteren bevorzugten Ausführungsform gilt, daß in diesem erfindungsgemäßen Verfahren in Stufe (b):
• die Erwärmung in der Hochdruckmikrowelle innerhalb von weniger als 5 Minuten, vorzugsweise weniger als 4 Minuten, erfolgt, und/oder
• die die Biomasse führende Vorrichtung der Hochdruckmikrowelle röhrenförmig ausgeführt ist; und/oder
• die die Biomasse führende Vorrichtung der Hochdruckmikrowelle eine lichte Weite von maximal 15 cm, vorzugsweise maximal 10 cm aufweist; und/oder
• die die Biomasse führende Vorrichtung innerhalb von weniger als 5 sec, vorzugsweise weniger als 2 sec, durch Kontaktieren mit einem Raum mit niedrigerem Gasdruck auf Umgebungsdruck reduziert/entspannt wird;
• der verwendete Mikrowellenaplikator eine Leistung im Bereich von 50 - 150 kW, und 800 -1100 MHz zeigt; und/oder
• im die Biomasse enthaltende Vorrichtungsteil der Hochdruckmikrowelle ein Druck zwischen 2 bar bis 30 bar, vorzugsweise 5 bar bis 25 bar, insbesondere 10 - 20 bar erreicht wird.

In einer weiteren bevorzugten Ausführungsform wird in diesem erfindungsgemäßen Verfahren vor Stufe (b) der Biomasse ein Hydrolyse-Katalysator zugesetzt, wobei dieser Katalysator vorzugsweise ausgewählt ist aus einer Mischung aus Redox- und Oxidationsmittel, vorzugsweise aus einer Mischung ausgewählt aus CuO/Cr₂O₃, ZnO/Cr₂O₃ oder CuO/Zn0.

In einer weiteren bevorzugten Ausführungsform erfolgt in diesem erfindungsgemäßen Verfahren der Vergärungsprozeß in Stufe (c) und/oder gegebenenfalls der Vergärungsperozess in Stufe (d)
• mikrobiologisch, enzymatisch oder sowohl mikrobiologisch als auch enzymatisch; und/oder
• bei einer Temperatur von zwischen 50 und 60 °C, vorzugsweise bei 55°C, oder bei einer Temperatur von zwischen 35 und 45 °C, vorzugsweise bei 40°C; und/oder
• der Vergärungsprozeß in einem Hochleistungsreaktor mit Faulraumbelastungen > 5 kg oTS/m³ Faulraum.

In einer weiteren bevorzugten Ausführungsform wird in diesem erfindungsgemäßen Verfahren die wasserhaltige lignoceellulosehaltige Biomasse ausgewählt aus:
Mais, Maissilage, Gras, Grassilage, Ganzpflanzengetreide, Silage aus Getreideganzpflanzen, Stroh, Dung oder auch ausgewählt aus Garten- oder Forstabfall wie Zweigen und Grünschnitt.

In einer weiteren bevorzugten Ausführungsform wird in diesem erfindungsgemäßen Verfahren die Biomasse zwischen den Stufen (a) und (b), zwischen den Stufen (d) und (b) und/oder zwischen den Stufen (c) und (b) auf Temperaturen bis zu 150 °C, vorzugsweise 140°C erwärmt, vorzugsweise durch Wärmeaustauschgeräte.

In einer weiteren bevorzugten Ausführungsform wird in diesem erfindungsgemäßen Verfahren die Biomasse zwischen den Stufen (b) und (c) von Temperaturen oberhalb von 150 °C, vorzugsweise oberhalb von 165°C abgekühlt auf Temperaturen unterhalb von 60 °C, vorzugsweise unterhalb von 56°C, vorzugsweise durch Wärmeaustauschgeräte.

Das erfindungsgemäße Verfahren kann sowohl als Batch-Verfahren als auch als kontinuierliches Verfahren durchgeführt werden. Besonders bevorzugt erscheint ein kontinuierliches Verfahren, da dabei insbesondere die Hochdruckmikrowelle sehr günstig zu betreiben ist, da so die Anzahl der Anfahrprozesses stärker minimiert ist.

Bevorzugt ist auch ein erfindungsgemäßes Verfahren, in dem
• in einer Stufe (a) die lignocellulosehaltige Biomasse durch ein Zerfaserungsgerät aufbereitet wird;
• in einer nachfolgenden Stufe (b) die optional zwischenbehandelte, aufbereitete lignocellulosehaltige Biomasse aus Stufe (a) zunächst in einer thermischen Hydrolyse (b1) in einer Hochdruckmikrowelle auf Temperaturen zwischen 150 und 250 °C erwärmt und hydrolysiert wird und anschließend in einem Druck-reduzierungsschritt (b2) der in der Hochdruckmikrowelle entstandene Druck in der Biomasse durch Kontaktieren der die Biomasse führenden Vorrichtung mit einem Raum mit niedrigerem Gasdruck reduziert wird;
• in einer nachfolgenden Stufe (c) die optional zwischenbehandelte hydrolysierte lignocellulosehaltige Biomasse aus Stufe (b) einem anaeroben Vergärungsprozess unterworfen wird.

Eine Abwandlung der Erfindung betrifft ein Verfahren zur Herstellung von Biogas oder Bioethanol aus wasser- und lignocellulosehaltiger Biomasse, in dem - ohne dass notwendigerweise zuvor eine Aufbereitungsstufe (a) mit einem Zerfaserungsgerät durchlaufen werden muss -
• in einer Stufe (d) lignocellulosehaltige Biomasse einem anaeroben Vorvergärungsprozess unterworfen wird;
• in einer nachfolgenden Stufe (b) lignocellulosehaltige Biomasse aus Stufe (d) in einer thermischen Hydrolyse (b1) in einer Hochdruckmikrowelle erwärmt und hydrolysiert wird; und
• in einer nachfolgenden Stufe (c) hydrolysierte lignocellulosehaltige Biomasse aus Stufe (b) einem anaeroben Vergärungsprozess unterworfen wird.

Natürlich kann in dieses Verfahren, beispielsweise vor der Stufe (d), aber auch nach den Stufen (b) oder (c), einmal oder mehrfach eine Aufbereitungsstufe (a) mit einem Zerfaserungsgerät integriert sein und das Verfahren kann auch einen oder mehrere Rezyklisierungsschritte (e) enthalten, in denen Biomasse aus dem anaeroben Vergärungsprozess der Stufe (c) erneut in einer Stufe (b) einer thermischen Hydrolyse (b1) in einer Hochdruckmikrowelle unterzogen und anschließend erneut einem anaeroben Vergärungsprozess in einer Stufe (c) unterworfen wird, wobei dies optional konsekutiv mehrfach durchgeführt wird. Auch die anderen bevorzugten Ausführungsformen der einzelnen Stufen des Verfahrens wie oben beschrieben sind auf diese alternative Abwandlung der Erfindung anzuwenden.

Ein weiterer Aspekt der Erfindung betrifft eine Verfahrensvorrichtung (Anlage) zur Herstellung von Biogas oder Bioethanol aus wasser- und lignocellulosehaltiger Biomasse mit mindestens folgenden durch zum Transport von Biomasse geeigneten Röhrensystemen verbundenen Vorrichtungen, wobei diese in Reihe miteinander oder über weitere Vorrichtungen aufeinander folgend verbunden sind:
i) Stoffannahmevorrichtung (S) zur Aufnahme, zum Sammeln und/oder zum Einspeisen der lignocellulosehaltigen Biomasse in die Anlage,
ii) Zerfaserungsgerät oder Extruder (Z) zur Zerkleinerung und Zerfaserung der lignocellulosehaltigen Biomasse und
iii) Erster Faulbehälter (F1) zur anaeroben Vergärung,
dadurch gekennzeichnet, dass zwischen Zerfaserungsgerät/Extruder (Z) und Erstem Faulbehälter (F1) als weitere Vorrichtung eine Mikrowellenanlage (MWA) mit Hochdruckmikrowelle eingefügt ist, die mit Extruder (Z) und Erstem Faulbehälter (F1) durch zum Transport von Biomasse geeignete Röhrensysteme direkt oder über weitere Vorrichtungen verbunden ist.

Die erfindungsgemäße Verfahrensvorrichtung (Anlage) ist zur Durchführung des erfindungsgemäßen Verfahrens geeignet, wobei die Stufe (a) im Zerfaserungsgerät oder Extruder (Z), die Stufe (b), insbesondere Stufe (b1) (gegebenenfalls mit Vorwärmschritten vor Stufe (b1) und Abkühlungsschritten nach Stufe (b1) oder (b2)) in der Mikrowellenanlage (MWA) und der erste anaerobe Vergärungsprozeß der Stufe (c) in einem ersten Faulbehälter (F1) durchgeführt wird.

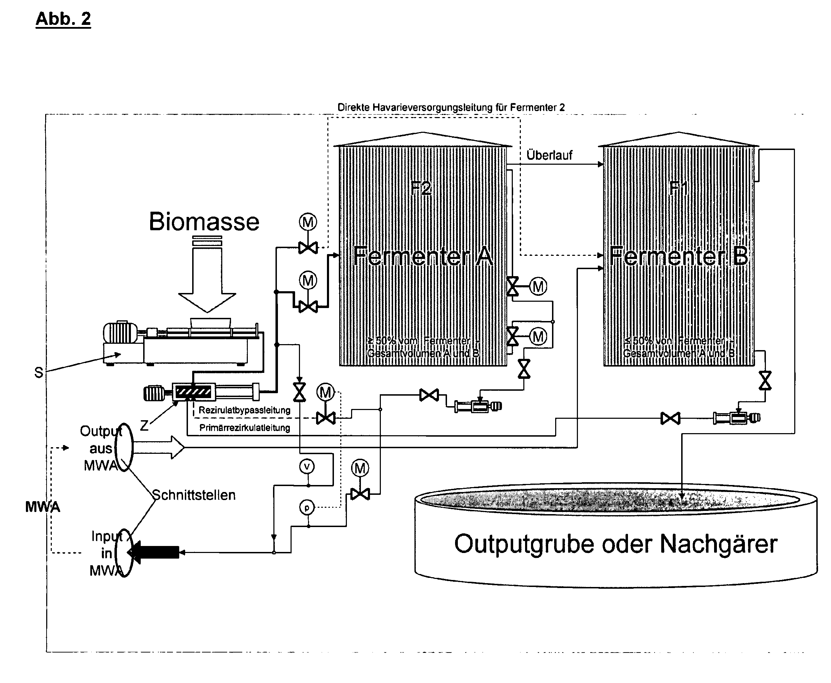

In bevorzugten Ausführungsformen des in der Verfahrensvorrichtung (Anlage) durchzuführenden Verfahrens durchläuft die in Stufe (a) aufbereitete/zerfaserte Biomasse zunächst einen anaeroben Vorvergärungsprozess (Stufe (d)), bevor sie zum ersten Mal der thermischen Hydrolyse (Stufe (b1)) unterworden wird. Je nach Prozess und Führung der die verschiedenen Vorrichtungen verbindenden, zum Transport von Biomasse geeigneten Röhrensysteme und der Pumprichtung der Biomasse in diesen Röhrensystemen (s. beispielsweise Abbildungen 2 bis 5) kann dieser Vorvergärungsprozeß bereits im Ersten Faulbehälter (F1) oder gegebenenfalls (auch) in einem oder mehreren Zweiten Faulbehältern (F2) durchgeführt werden.

In weiteren bevorzugten Ausführungsformen des in der Verfahrensvorrichtung (Anlage) durchzuführenden Verfahrens durchläuft die Biomasse aus dem anaeroben Vergärungsprozess der Stufe (c) in einem Rezyklisierungsschritt (e) erneut in einer Stufe (b) eine thermische Hydrolyse (b1) und anschließend erneut einen anaeroben Vergärungsprozess in einer Stufe (c), wobei dieser Rezyklisierungsschritt (e) gegebenfalls konsekutiv mehrfach durchgeführt wird und gegebenenfalls auch die Stufe (a) mit einem Zerfaserungsgerät umfaßt. Je nach Prozess und Führung der die verschiedenen Vorrichtungen verbindenden, zum Transport von Biomasse geeigneten Röhrensysteme und der Pumprichtung der Biomasse in diesen Röhrensystemen (s. beispielsweise Abbildungen 2 bis 5) kann dieser oder können diese anaeroben Vergärungsprosse (Stufe (c)) des Rezyklisierungsschritts (e) im Ersten Faulbehälter (F1) oder gegebenenfalls (auch) in einem oder mehreren Zweiten Faulbehältern (F2) durchgeführt werden.

In einer bevorzugten Ausführungsform der erfindungsgemäßen Vorrichtung gilt,
dass zwischen dem Extruder (Z) und der Mikrowellenamlage (MWA) mindestens ein zweiter Faulbehälter (F2) zur anaeroben Vergärung angeordnet ist, der mit dem Extruder (Z) und der Mikrowellenanlage (MWA) durch zum Transport von Biomasse geeigneten Röhrensysteme direkt oder über weitere Vorrichtungen verbunden ist; und/oder
dass der Erste Faulbehälter (F1) mit der Mikrowellenanlage (MWA) durch weitere zum Transport von Biomasse geeignete Röhrensysteme direkt und/oder über weitere Vorrichtungen so verbunden ist, dass Biomasse vom Ersten Faulbehälter (F1) zur Mikrowellenanlage (MWA) transportiert wird; oder
dass der Erste Faulbehälter (F1) mit der Mikrowellenanlage (MWA) durch weitere zum Transport von Biomasse geeignete Röhrensysteme über den Extruder (Z) und/oder mindestens einen Zweiten Faulbehälter (F2) und gegebenenfalls weitere Vorrichtungen so verbunden ist, dass Biomasse vom Ersten Faulbehälter (F1) zur Mikrowellenanlage (MWA) transportiert wird.

Eine weitere Abwandlung der Erfindung betrifft eine Verfahrensvorrichtung (Anlage) zur Herstellung von Biogas oder Bioethanol aus wasser- und lignocellulosehaltiger Biomasse mit mindestens folgenden durch zum Transport von Biomasse geeigneten Röhrensystemen verbundenen Vorrichtungen, wobei diese in Reihe miteinander oder über weitere Vorrichtungen aufeinander folgend verbunden sind und die Verfahrensvorrichtung nicht zwangsläufig, aber optional ein Zerfaserungsgerät/Extruder (Z) enthält:
• Stoffannahmevorrichtung (S) zur Aufnahme, zum Sammeln und/oder zum Einspeisen der lignocellulosehaltigen Biomasse in die Anlage,
• Zweiter Faulbehälter (F2) zur anaeroben Vergärung,
dadurch gekennzeichnet, dass nach dem zweiten Faulbehälter (F2) als weitere Vorrichtung eine Mikrowellenanlage (MWA) mit Hochdruckmikrowelle eingefügt ist, die mit dem Zweiten Faulbehälter (F2) und gegebenenfalls einem Ersten Faulbehälter (F1) durch zum Transport von Biomasse geeignete Röhrensysteme direkt oder über weitere Vorrichtungen verbunden ist.

Der folgende Abschnitt zeigt verschiedene Ausführungsformen der Erfindung und ist keinesfalls als beschränkend zu verstehen.

### BEISPIELE

### Beispiel 1:

### Prinziplösung des Verfahrens

### 1. Vorbereitung des Rohstoffsubstrates

Die lignocellulosehaltige Biomasse für den Naß- oder Trockenfermentationsprozess der Biogasanlage und/oder für die Bioethanolproduktion mit Wassergehalten von ≥ 30% wird bei der Ernte auf 1 bis 4 cm Schnittlänge, bzw. möglichst klein gehäckselt. Für die Verarbeitung von langfasrigen Biomassen wird dem Extruder ein Vorzerkleinerer vorgeschaltet. Das Häckselgut wird unmittelbar danach als Frischmasse oder später als Silage in die erfindungsgemäße Anlage, in der das erfindungsgemäße Verfahren durchgeführt werden kann, eingebracht.

### 2. Aufschluss von lignocellulosehaltiger Biomasse

### a) durch Zerfaserung

Die gehäckselte Biomasse, frisch geerntet oder als Silage, wird über einen Dosiermischer mit oder ohne Vorzerkleinerer in einen Ein- oder Doppelschneckenextruder geleitet. Im Extruder wird die morphologische Makrostruktur der Biomasse zerstört, das heißt der Zellsaft wird teilweise ausgepresst und die feste Biomasse zerfasert.

### Der Vorteil des Zufaserungsgerät-/Extrudereinsatzes:

Aus der strukturierten Biomasse wird ein pumpfähiges, breiiges, hydrophiles Substrat mit stark vergrößerter, für Mikroorganismen und Enzyme leichter angreifbarer Oberfläche gebildet. Während des Extruderdurchlaufes wird das Substrat - überwiegend durch Scherkräfte und Druck - auf etwa 40°C bis 70°C erwärmt.

Außerdem bewirkt die Zerfaserung der Biomasse im vorgeschalteten Extruder eine Verbesserung der Substrathomogenitat, eine Erhöhung des Saug- und Wasserbindungsvermögens, eine Verringerung der Schwimmschichtbildung und eine intensivere, gleichmäßigere thermische Hydrolyse im nachfolgen Schritt (b).

Vom Extruder aus wird das Substrat für die thermische Hydrolyse in eine Hochdruckmikrowelle gepumpt. Auf dieser Wegstrecke erfolgt mit Hilfe von Prozessenergie aus der Biogasverstromung vorzugsweise eine zusätzliche Substraterwärmung auf bis zu 140 bis 160° C. Dies ist unter bestimmten Umständen energetisch günstiger als die Erwärmung alleine mit Hilfe der Hochdruckmikrowelle.

### b) Mikrowelle

In der Hochdruckmikrowelle wird das Substrat hydolysiert. Dabei wird das Substrat, die lignocelluslosehaltige Biomasse, in kurzer Zeit erhitzt, vorzugsweise (etwa in zwischen 10 bis 120 sec) auf 160 bis 220° C. Dabei baut sich - optional auch durch eine Verjüngung des Transportrohrs - ein überatmosphärischer Druck, insbesondere ein Druck von 5 bis 25 bar auf. Anschließend erfolgt optional und vorzugsweise eine schlagartige Entspannung auf Umgebungsdruck über ein regelbares Schlagventil. Durch die Temperaturerhöhung und mit dem Wasser der wasserhaltigen Biomasse findet in der Mikrowelle oder am Mikrowellenapplikator, eine thermische Hydrolyse unter Druck statt. Durch die thermische Hydrolyse in der Mikrowelle löst sich der Lignocellulosekomplex. Die optionale und bevorzugte schlagartige Entspannung führt zu einer weiteren Auflösung und zum Zerreißen der noch vorhandenen Strukturen und Komplexverbindungen. Die hydrophilen Eigenschaften des Substrates werden erheblich verstärkt. Die Hemicellulose geht zu einem hohen Anteil in Lösung und die Cellulose bleibt mit dem Lignin nur noch locker verbunden. Die amorphen Stellen der Cellulose sind für Mikroorganismen und Enzyme relativ leicht angreifbar und die Cellulose ist auch teilweise schon veflüssigt und verzuckert, was die Produktion von Bioethanol erleichtert.

Dabei steigt die Effektivität der thermischen Hydrolyse weiter an, wenn der produktführende Teil der Hochdruckmikrowelle, insbesondere das Biomasse transportierende Rohr, lediglich einen geringen Durchmesser aufweist, insbesondere einen Durchmesser von maximal 10 cm, da dadurch die um das Rohr angeordneten Magnetrone die Biomasse von allen Seiten erreichen können und sie damit sowohl von allen Seiten erreicht und erhitzt werden kann. Dadurch steigt die Temperatur im Substrat/der Biomasse in kürzester Zeit auf die konzipierte Höhe.

Schnelle Erwärmung bedeutet aber auch schnelle Verzuckerung bei geringem Zuckerzerfall. Auf diese Weise werden günstige Lebensbedingungen für die Mikroorganismen in Schritt (c) geschaffen und hohe Biogas- bzw. Ethanolausbeuten erreicht.

Bestimmte Parameter können für die thermische Hydrolyse besonders günstig sein:

| **Parameter** | **Größenordnung** | **Bevorzugte Größenordnung** | |
|---|---|---|---|
| | | **Biogas** | **Ethanol** |
| **Druck** | 5 - 25 bar | 15 bar | 20 bar |
| **Temperatur** | 160 - 220° C | 160 - 170° C | 180 - 220° C |
| **Verweilzeit** | 10 - 220 sec | 30 - 60 sec | 30 - 60 sec |

Vor dem Schritt (b) kann zur Stimulierung des Hydrolyseprozesses bei bestimmten Biomassen ein Katalysator (Redox-/Oxidationsmittel) hinzugegeben werden. In solch einem Fall durchläuft die Biomasse auf dem Weg vom Zerfaserer/Extruder zur Hochdruckmikrowelle einen Mischer mit Katalysatoreneinspritzdüsen.

### 3. Vergärung

Im Schritt (c) der Gärung werden Mikroorganismen und Enzyme zur Anwendung gebracht um Biogas und Bioethanol herzustellen. Dabei kann das erfindungsgemäße Verfahren auch als Kombination mit Hochleistungsreaktoren für die Biogas- und/oder Bioethanolgewinnung durchgeführt werden. Unter Hochleistungsreaktoren werden solche mit Faulraumbelastungen > 5 kg oTS/m³ Faulraum betrachtet. Hier wirken sich die vorbereitenden Vorteile der Schritte (a) und (b) aus und resultieren in folgenden Vorteilen, insbesondere für die Biogasproduktion:
- Der Fermentationsprozess, insbesondere der Trockenfermentationsprozess, verkürzt sich auf 10 bis 20 Tage.
- Die Biomasseausnutzung erhöht sich auf 65 - 80 % der Trockenmasse.
Vorteile für die Bioethanolproduktion
- Die Zuckerausbeuten steigen auf 75 bis 85 % des Polysacharidgehaltes in der Trockenmasse.
- Der Anteil an Zuckerverfallsprodukten in Form der Inhibitoren Furfural und 5-Hydroxymethylfurfural bleibt konstant niedrig bei maximal 2,5 %.
- Die Ethanolausbeuten steigen.

### Beispielhafte technische Lösung das erfindungsgemäßen Verfahrens

Neben den bereits benannten Prozessparametem zu Druck, Temperatur und Verweilzeiten wird der Mengendurchfluss auf 0,5 m³/h bis 10 m³/h, im Sonderfall auch größer, festgelegt.

### Abbildungen:

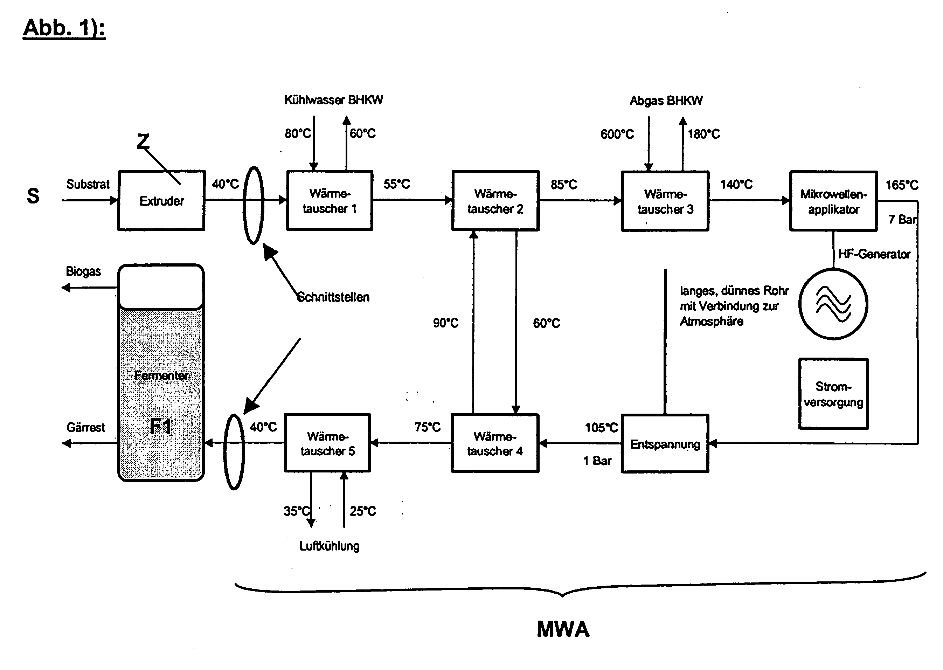

- **Abbildung 1)**: zeigt eine bevorzugte Ausführungsform der Erfindung, insbesondere in detaillierter Form eine Mikrowellenanlage (MWA) zum Einsatz im erfindungsgemäßen Verfahren oder Vorrichtung.
- **Abbildung 2)**: zeigt eine bevorzugte Ausführungsform (Zweistufige Vergärung) der Erfindung, in der systematisch erfindungsgemäße Vorrichtungen geeignet zur Durchführung des erfindungsgemäßen Verfahrens, der Stufen (a), (b), (c) und evt. (d) und (e) gezeigt werden, wobei die Mikrowellenanlage (MWA) aus Abbildung 1 über die "Schnittstellen" schematisch integriert ist.

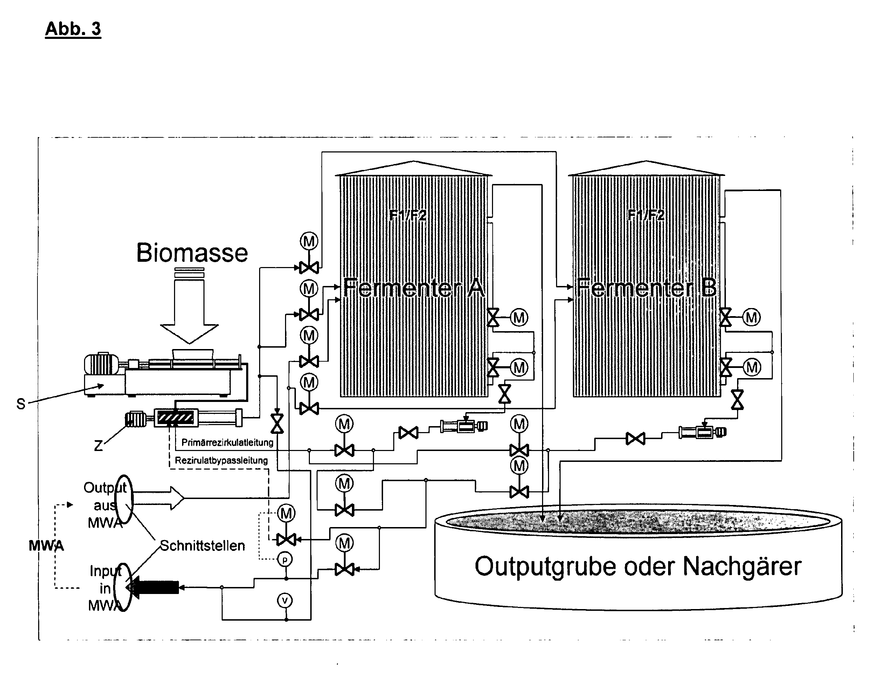

- **Abbildung 3)**: zeigt eine weitere bevorzugte Ausführungsform (Parrallelbetrieb) der Erfindung, in der systematisch erfindungsgemäße Vorrichtungen geeignet zur Durchführung des erfindungsgemäßen Verfahrens, der Stufen (a), (b), (c) und evt. (d) und (e) gezeigt werden, wobei die Mikrowellenanlage (MWA) aus Abbildung 1 über die "Schnittstellen" schematisch integriert ist.

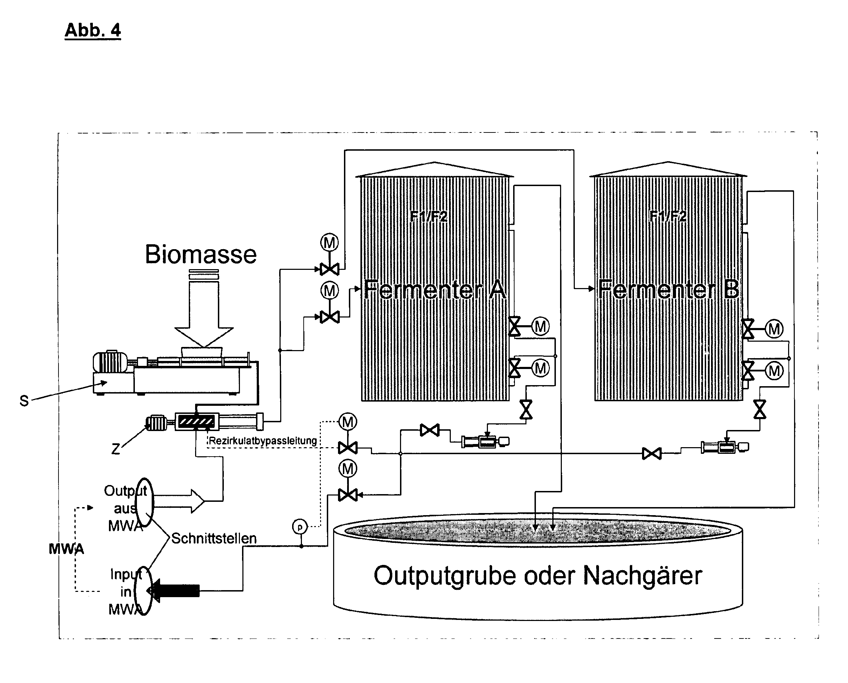

- **Abbildung 4)**: zeigt eine weitere bevorzugte Ausführungsform (Rezirkulataufbereitung) der Erfindung, in der systematisch erfindungsgemäße Vorrichtungen geeignet zur Durchführung des erfindungsgemäßen Verfahrens, der Stufen (a), (b), (c) und evt. (d) und (e) gezeigt werden, wobei die Mikrowellenanlage (MWA) aus Abbildung 1 über die "Schnittstellen" schematisch integriert ist.

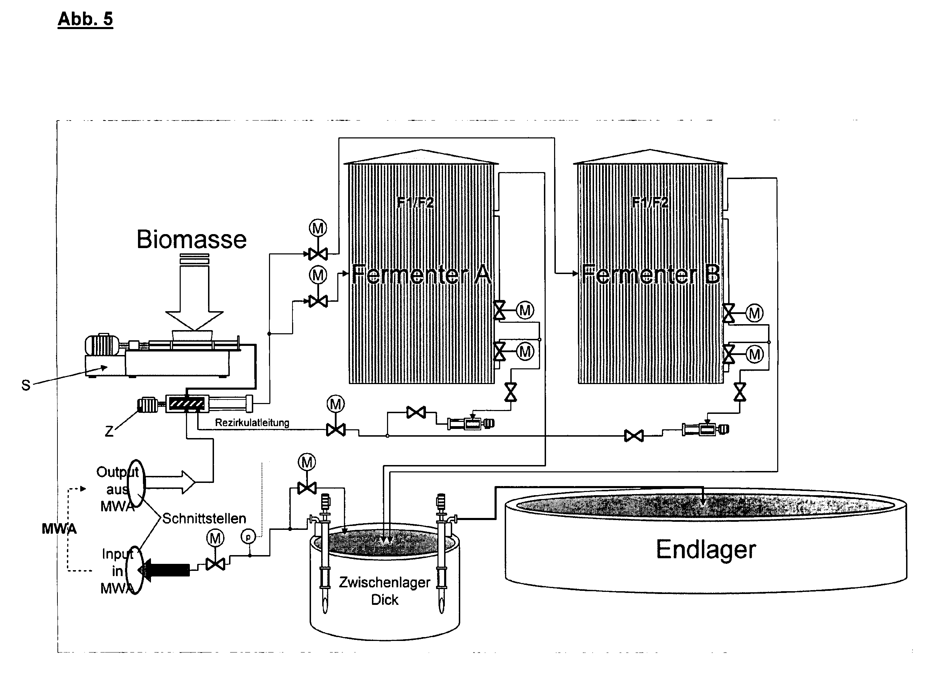

- **Abbildung 5)**: zeigt eine weitere bevorzugte Ausführungsform (Intensive Gärstoffverwertung) der Erfindung, in der systematisch erfindungsgemäße Vorrichtungen geeignet zur Durchführung des erfindungsgemäßen Verfahrens, der Stufen (a), (b), (c) und evt. (d) und (e) gezeigt werden, wobei die Mikrowellenanlage (MWA) aus Abbildung 1 über die "Schnittstellen" schematisch integriert ist.

### Erläuterungen zu Abbildung 1:

Abbildung 1) zeigt eine bevorzugte Ausführungsform der Erfindung, in der systematisch der Ablauf des erfindungsgemäßen Verfahrens und eine für dieses Verfahren geeignete Anlage zur Biogasherstellung gezeigt wird. Insbesondere zeigt die Abbildung die Mikrowellenanlage (MWA). Insoweit bezieht sich Abbildung 1 insbesondere auch auf Stufe (b) des erfindungsgemäßen Verfahrens und erläutert diese bzw. die optionalen Wärme-Vorbehandlungssschritte vor der thermischen Hydrolyse in der Hochdrucmikrowelle in Stufe (b1) sowie die optionalen Abkühlungsschritte nach der bevorzugten, optionalen Entspannung (Druckreduzierungsschritt) in Stufe (b2). So wird nach dem Zerfaserungsschritt im Zerfaserungsgerät/Extruder in Stufe (a) und evtl. einem optionalen Vorvergärungsschritt (Stufe (d); nicht in Fig. 1 abgebildet) die Biomasse durch verschiedene Wärmeaustauscher erwärmt, bis die Biomasse im Mikrowellenaplikator einer thermischen Hydrolyse in dem Hochdruckmikrowellenapplikator unterzogen wird (Stufe b1). In weiteren bevorzugten Ausführungsformen (gezeigt in Abbildungen 2 bis 5), die sich auf die Abfolge der Stufen (a), (c), (d) und auch Rezyklisierungsschritt (e) auch im örtlichen und zeitlichen Bezug zu Stufe (b) beziehen, wird über die Schnittstellen in den Abbildungen schematisch die beispielhaft in Abbildung 1 gezeigte Mikrowellenanlage (MWA; Stufe b) in die gezeigten beispielhaften erfindungsgemäßen Vorrichtungen integriert.

Das Substrat, die lignocellulosehaltige Biomasse - in einem Falle mit Dung versetztes Stroh, ansonsten diverse Biomassen - wird in einer Stoffannahmevorrichtung (S) (nicht explizit abgebildet) gesammelt und über einen Schneckenförderer (nicht abgebildet) in das Zerfaserungsgerät oder den Extruder (Z) eingebracht. Der Extruder (Z), dem ein Magnetabscheider oder ein Vorhächsler vorgeschaltet sein kann, zerkleinert und homogenisiert die Biomase. Dabei erwärmt sich die Biomasse durch den Aufschluss auf ca. 40 °C. Eine Dickstoffpumpe bringt die Biomasse in die Mikrowellenanlage (MWA), gegebenenfalls unter Zugabe von Rezirkulat (flüsigerer Biomasse, die bereits einem anaeroben Vergärungsprozeß unterworfen war). Dabei wird die Biomasse unter Durchlauf verschiedener Wärmetauscher, die aus Gründen der Energieersparnis die Biomasse bereits vorerwärmen, zu einem Mikrowellenapplikator (Hochdruckmikrowelle) mit einem Mikrowellengenerator gepumpt. Die Erwärmung der Biomasse in den Wärmetauschern beruht auf verschiedenen Prinzipien wie Gegenstrom oder Wärmeaustausch und verwendet zum Teil die Energie aus der Verstromung des Biogases (Abgaswärmetausch). Der Mikrowellengenerator, in dem die Stufe (b1) des erfindungsgemäßen Verfahrens durchgeführt wird, zeigt eine Leistung von 100kW/915MHz und der Aplikator eine Leistung von 75kW/915MHz. Das Rohr, das die Biomasse führt und den Mikrowellenapplikator durchläuft hat einen Durchmesser 40 mm und in einem anderen Fall 100 mm oder zwischen 40 und 100 mm. Durch Verringerung des Querschnitts wird weiter der Druck erhöht. Im Applikator werden für einen Zeitraum von 10-220 sec, insbesondere 30-60 sec. Mikrowellen zugeführt. Durch diese Maßnahmen steigt der Druck im Applikator auf Werte zwischen 5 und 15 bar, in Ausnahmefällen - insbesondere bevorzugt bei Bioethanol - auf bis zu 25 bar an.

Die dort erreichten Temperaturen können auf Werte zwischen 160 -220°C reguliert werden, wobei bei der Biogas-Herstellung 160 - 170° C und bei der Bioethanolherstellung 180 - 220° C bevorzugt sind. Über ein Entspannungsventil, das zur Durchführung des optionalen Druckreduzierungsschritts (b2) dient und das als Schlagventil ausgeführt und mit der Atmosphäre verbunden ist, wird der Druck im Biomasse führenden Rohr schlagartig auf atmosphärischen Druck zurückgeführt. Alle diese Massnahmen (Mikrowellenerhitzung, thermische Hydrolyse, Druckerhöhung und der optionale, aber bevorzugte schlagartige Druckverlust/Druckentspannung) führen zu einer Auflösung des Lignocellulosekomplexes, da sich die Verklebung von Hemicellulose, Lignin und Cellulose löst und die noch vorhandenen Strukturen und Komplexverbindungen durch die schlagartige Entspannung zerreißen. Es entsteht eine feste und eine flüssige Fraktion. In der flüssigen Fraktion sind überwiegend die Zucker der Hemicellulose gelöst. Die feste Fraktion besteht aus Cellulose und Lignin. Die Cellulose ist im amorphen Teil geschwächt und in Teilen sogar verflüssigt und verzuckert. Das Lignin lässt sich durch Lösemittel (Natronlauge oder Ethanol) von der Cellulose trennen und als gesondertes Produkt handeln oder für die Gewinnung von Prozessenergie nutzen. In besonderen Fällen kann der Biomasse vor dem Mikowellenapplikator ein Katalysator über einen Mischer mit Katalysatoreneinspritzdüsen hinzugegeben werden. Die Biomasse wird dann weiter unter Passieren verschiedener Wärmeaustauscher, die (teilweise im Gegenstrom mit den Wärmetauschern vor dem Mikrowellenapplikator) die Biomasse auf Temperaturen unter 45 °C abkühlen, in einen Fermenter/Biogasreaktor/Ersten Faulbehälter (F1) eingebracht. In einer bevorzgten Ausführungsform handelt es sich um einen Hochleistungsreaktor für die Biogas- und/oder Bioethanolgewinnung mit einer Faulraumbelastungen > 5 kg oTS/m³ Faulraum. Die bevorzugte Temperatur im Reaktor hängt von der Auswahl der verwendeten Mikroorganismen ab und beträgt 55 °C bei thermophilen, bzw. 35 - 45°C bei mesophilen Mikroorganismen.
In besonderen Fällen kann die Biomasse vom Extruder (Z) zunächst in einen Zweiten Faulraum (F2) gepumpt und von diesem dann in die Mirkrowellenanlage (MWA) eingebracht werden.

### Erläuterung zu Abbildung 2:

Abbildung 2) zeigt eine bevorzugte Ausführungsform (Zweistufige Vergärung) der Erfindung, in der systematisch erfindungsgemäße Vorrichtungen geeignet zur Durchführung des erfindungsgemäßen Verfahrens, der Stufen (a), (b), (c) und evtl. (d) und (e) gezeigt werden, wobei die Mikrowellenanlage (MWA), wie sie beispielhaft in Abbildung 1 erläutert wurde, über die "Schnittstellen" schematisch integriert ist. Das Schema für die sequentielle Fermentervergärung hat eine bessere Vergärung infolge einer intensiveren nacheinander geschalteten Fermenterarchitektur zum Ziel. Dabei wird der Fermenter A (F2) deutlich größer ausgelegt als der Fermenter B (F1). Durch die Trennung der Einzelfermenter und deren unterschiedlichen Substratbeschickungsarten, kann das mikrobiologische Milieu in jeden Fermenter angepasst werden (z.B pH Wertanpassung, etc.). Somit erfolgt eine Vorvergärung (Stufe d) in Fermenter A (F2) und die Nachvergärung (Stufe c) in Fermenter B (F1). Der Biomasse-Eintrag (über Stufe a) geht primär in den Fermenter A (F2), wo die Biomasse zum Teil schon von den Mikroorganismen aufgeschlossen wird. Die permanent fördernde Rezirkulatleitung von Fermenter A (F2) ist für die Beschickung der Hochdruckpumpe der Aufschlussanlage (MWA) vorgesehen. Eine Rezirkulatbypassleitung reguliert dabei den Volumenstromüberschuss und leitet diesen in die Rachenpumpe (Z) der zentralen Einbringanlage ab. Sie kann aber auch direkt in Fermenter A (F2) zurückgeführt werden (Abhängig vom Gesamtanlagenaufbau). Die Regelung erfolgt über eine Druckmessung, welche gewährleisten soll, dass ein Eingangsdruck von rund 2 bis 4 bar an der Hochdruckpumpe anliegt. Parallel zum primären Rezirkulateintrag aus Fermenter A ist aber auch ein Knotenpunkt für den Frischbiomasseeintrag in den Aufschlussanlageneingang (MWA; Schnittstelle) vorgesehen. Dieser soll von Hand betätigt ausreichend frische Biomasse zusätzlich eintragen können. Diese Maßnahme dient in erster Linie zur Parameteroptimierung und sollte vorzugsweise mit entsprechenden Volumenmesseinrichtungen versehen werden.
Der aufgeschlossene Materialmix aus Fermenter A (F2) und der Biomasseleitung wird nach dem Verlassen der Stufe (b) in der Aufschlussanlage (MWA) in Fermenter B (F1) (in Stufe c) weitervergoren. Die Rezirkulatleitung aus Fermenter B (F1) hat die Hauptfunktion zur Anmaischung der Biomasse in der Rachenpumpe (Z). Das endgültig vergorene Material verlässt die Biogasanlage über Fermenter B (F1) zum Zwischenlager, Endlager etc.. Der Fermenter A (F2) ist mit Fermeter B (F1) über ein Überlauf verbunden, um ein Überlaufen von Fermenter A (F2) zu verhindern. Der Überlauf befindet sich über dem Mischleitungsabgang, welcher den Rezirkulatstrom aus dem oberen und unteren Fermenterbereich vermengt. Somit kann zielgerichteter der aufgeschwommene Feststoffanteil in die Rezirkulatleitung der Aufschlussanlage (MWA) zurückgeführt werden. Für den Fall einer Havarie an der Aufschlussanlage, befindet sich eine direkte Havarieleitung für Fermenter B. Sie wird im Havariefall zum Füttern von Fermenter B benutzt und ist bei Normalbetrieb geschlossen.

### Erläuterung zu Abbildung 3:

Abbildung 3) zeigt eine weitere bevorzugte Ausführungsform (Parrallelbetrieb) der Erfindung, in der systematisch erfindungsgemäße Vorrichtungen geeignet zur Durchführung des erfindungsgemäßen Verfahrens, der Stufen (a), (b), (c) und evt. (d) und (e) gezeigt werden, wobei die Mikrowellenanlage (MWA), die in Abbildung 1 beispielhaft beschrieben wurde, über die "Schnittstellen" schematisch integriert ist.

Der komplette Parallelbetrieb für die Zuführung der Biomasse zur Mikrowellenanlage (MWA) hat den Vorteil, dass beide Fermenter mit unterschiedlich aufgeschlossenen Material versorgt werden können. Damit ist ein direkter Vergleich von unterschiedlichen Substrataufbereitungsstufen, welche in die Fermenter A und B eingebracht werden, sehr gut möglich da sich die daraus resultierenden unterschiedlichen Gaserträge gut bilanzieren lassen. Die Einbring- und Austragstechnik beider Fermenter ist dupliziert und somit komplett getrennt steuerbar. Mit diesem Aufbau ist es möglich unter Zuhilfenahme einer intelligenten Steuerung beiden Fermentern gezielt unterschiedliche Milieubedingungen zuzuweisen. Ähnlich wie die Rezirkulatleitung aus der sequenzielle Fermentervergärung wird an beiden Fermentern eine Mischleitung angebracht, um auch hier den Feststoffanteil im Rücklaufstrom besser anpassen zu können.
Durch ein wechselseitiges Fördern der Rezirkulatpumpen, ist es möglich, einen dauerhaften Substratstrom für die Aufschlussanlage (MWA; Stufe (b)) aufbauen zu können, ohne dabei in die direkte Fütterstrecke eingreifen zu müssen. Dafür muss allerdings die Rezirkulatleitung sehr komplex mit Motorschiebern verknüpft werden.
Die Outputleitung der Aufschlussanlage wird direkt an den/die Fermenter A/B (F2) angeschlossen. Damit ist ein kontinuierliche aufgeschlossener Stoffstrom in jeweils dem vorher zugewiesenen Fermenter möglich. Die Zuführung der Aufschlussanlage wird ähnlich gesteuert wie beim sequentiellen Betrieb (druckgesteuerte Abführung des Rezirkulatvolumenstromesüberschusses in die Rachenpumpe).
Auch bei dieser Variante ist eine Direktbiomasseleitung vorgesehen, um die frische Biomasse direkt aufschließen zu können. Das Bilanzierungsprinzip ist mit dem der sequentiellen Anlage identisch.

### Erläuterung zu Abbildung 4:

Abbildung 4) zeigt eine weitere bevorzugte Ausführungsform (Rezirkulataufbereitung) der Erfindung, in der systematisch erfindungsgemäße Vorrichtungen geeignet zur Durchführung des erfindungsgemäßen Verfahrens, der Stufen (a), (b), (c) und evt. (d) und (e) gezeigt werden, wobei die Mikrowellenanlage (MWA), die in Abbildung 1 beispielhaft gezeigt wurde, über die Schnittstellen schematisch integriert ist.
Bei dem Rezirkulataufbereitungsschema wird der Zufluss zur Mikrowellenanlage (MWA) komplett aus Rezirkulat gespeist zur weitestgehenden Rezirkulatverdünnung. Dies kommt in Betracht bei Anlagen mit hohen Feststoffgehalt (Trockenfermentationsanlagen). Wenn der Rezirkulatrückstrom in Trockenfermentationsanlagen einen sehr hohen Feststoffanteil aufweist, hat das zur Folge, dass im zunehmenden Maße unvergorenes Material in den Rezirkulatstrom gelangt. Diese Ausführungsform der Vorrichtung wirkt diesem Phänomen entgegen. Ziel ist es, den angegorenen Substratstrom weiter aufzuschließen um einer Feststoffanreicherung entgegenzuwirken.
Die Zufuhr in die Mikrowellenanlage (MWA; Stufe (b)) wird wechselseitig durch die Rezirkulatpumpen eingebracht. Der Rezirkulatvolumenstromenüberschuss wird wieder druckgesteuert in die Rachenpumpe (Z) abgeführt. Entnommen wird das Rezirkulat wieder aus einer Mischleitung am/an den Fermenter/n A/B (F2;F1). Der verdünnte aufgeschlossene Outputstrom aus der Aufschlussanlage (MWA) wird in die Rachenpumpe (Z) gefördert und hat primär die Aufgabe der Anmaischung der Frischbiomasse. Bei Nichtbetrieb des Extruders (Z) wird der aufgeschlossene Stoffstrom in den Fermenter (F1) gefördert, aus welchen gerade Rezirkulat entnommen wird. Ein Wechsel der Rezirkuatförderung im Stundentakt wäre dabei sinnvoll.
Bei einer Havarie der Aufschlussanlage wird das Rezirkulat kompett über den Bypass geleitet.
Bei diesem Fließschema wird nicht der Frischbiomassestrom in die Aufschlussanlage (MWA) eingebracht. Dies hat den Vorteil, dass das erfindungsgemäße Verfahren durch geringfügigere Abwandlungen in bestehenden Anlagen durchgeführt und eingeführt werden kann.

### Erläuterung zu Abbildung 5:

Abbildung 5) zeigt eine weitere bevorzugte Ausführungsform (Intensive Gärstoffverwertung) der Erfindung, in der systematisch erfindungsgemäße Vorrichtungen geeignet zur Durchführung des erfindungsgemäßen Verfahrens, der Stufen (a), (b), (c) und evt. (d) und (e) gezeigt werden, wobei die Mikrowellenanlage (MWA), die beispielhaft in Abbildung 1 abgebildet ist, über die Schnittstellen schematisch integriert ist.
Die erfindungsgemäße Vorrichtung mit intensiverer Gärreststoffverwertung beinhaltet die teilweise Rückführung von "dicken" Reststoffsubstrat in die Aufschlussanlage (MWA) und damit zurück in die Fermenter A/B (F1). Dadurch kann unter anderem ein gewisser Anteil von ausgeschwemmten Spurenelementen in die Biogasanlage zurückgeführt werden. Der Output der Aufschlussanlage (MWA) wird in die Rachenpumpe (Z) eingetragen und von dort mit der Frischbiomasse wieder in die Fermenter A/B (F1,F2) eingebracht. Die schon bestehende Anlagenarchitektur von vorhanden Biogasanlagen bleibt durch die Integration der Aufschlussanlage relativ unbeeinflusst, so dass diese erfindungsgemäße Vorrichtung eine einfache Einfügung des erfindungsgemäßen Verfahrens in bestehende Anlagen erlaubt.

## Patentansprüche

1. Verfahren zur Herstellungvon Biogas oder Bioethanol aus wasser- und lignocellulosehaltiger Biomasse, **dadurch gekennzeichnet, dass**
• in einer Stufe (a) lignocellulosehaltige Biomasse durch ein Zerfaserungsgerät aufbereitet wird;
• in einer nachfolgenden Stufe (b) aufbereitete lignocellulosehaltige Biomasse aus Stufe (a) in einer thermischen Hydrolyse (b1) in einer Hochdruckmikrowelle erwärmt und hydrolysiert wird; und
• in einer nachfolgenden Stufe (c) hydrolysierte lignocellulosehaltige Biomasse aus Stufe (b) einem anaeroben Vergärungsprozess unterworfen wird.

2. Verfahren nach Anspruch 1, **dadurch gekennzeichnet, dass** zerfaserte lignocellulosehaltige Biomasse aus Stufe (a) vor Durchlaufen der Stufe (b) in einer Stufe (d) einem anaeroben Vorvergärungsprozess unterworfen wird.

3. Verfahren nach Anspruch 1 oder 2, **dadurch gekennzeichnet, dass** in einem Rezyklisierungsschritt (e) Biomasse aus dem anaeroben Vergärungsprozess der Stufe (c) erneut in einer Stufe (b) einer thermischen Hydrolyse (b1) in einer Hochdruckmikrowelle unterzogen und anschließend erneut einem anaeroben Vergärungsprozess in einer Stufe (c) unterworfen wird.

4. Verfahren nach Anspruch 3, **dadurch gekennzeichnet, dass** der Rezyklisierungsschritt (e) gemäß Anspruch 3 konsekutiv mehrfach durchgeführt wird.

5. Verfahren nach einem der Ansprüche 3 oder 4, **dadurch gekennzeichnet, dass** vor dem Rezyklisierungsschritt (e) gemäß Anspruch 3, oder vor einem, mehreren oder allen der Rezyklisierungsschritte (e) gemäß Anspruch 4 die Biomasse aus dem anaeroben Vergärungsprozess der Stufe (c) vor Zuführung zur Stufe (b) in einer Stufe (a) durch ein Zerfaserungsgerät aufbereitet wird.

6. Verfahren nach Anspruch 1 oder 5, **dadurch gekennzeichnet, dass** die Zerfaserung in Stufe (a) als Extrusion mit einem Extruder ausgelegt ist, insbesondere mit einem als Einfach- oder Doppelschneckenextruder bzw. Doppelkammerextruder ausgeführten Extruder erfolgt.

7. Verfahren nach Anspruch 1 bis 6, **dadurch gekennzeichnet, dass** in Stufe (b)
• Biomasse in der thermischen Hydrolyse (b1) in der Hochdruckmikrowelle auf Temperaturen zwischen 150 und 250 °C erwärmt und hydrolysiert wird; und/oder
• anschließend in einem Druckreduzierungsschritt (b2) der in der Hochdruckmikrowelle entstandene Druck in der Biomasse durch Kontaktieren der die Biomasse führenden Vorrichtung mit einem Raum mit niedrigerem Gasdruck reduziert wird.

8. Verfahren nach Anspruch 1 oder 7, **dadurch gekennzeichnet, dass** in Stufe (b)
• die Erwärmung in der Hochdruckmikrowelle innerhalb von weniger als 5 Minuten, vorzugsweise weniger als 4 Minuten, erfolgt, und/oder
• die die Biomasse führende Vorrichtung der Hochdruckmikrowelle röhrenförmig ausgeführt ist; und/oder
• die die Biomasse führende Vorrichtung der Hochdruckmikrowelle eine lichte Weite von maximal 15 cm, vorzugsweise maximal 10 cm aufweist; und/oder
• die die Biomasse führende Vorrichtung innerhalb von weniger als 5 sec, vorzugsweise weniger als 2 sec, durch Kontaktieren mit einem Raum mit niedrigerem Gasdruck auf Umgebungsdruck reduziert/entspannt wird;
• der verwendete Mikrowellenaplikator eine Leistung im Bereich von 50 -150 kW, und 800 - 1100 MHz zeigt; und/oder
• im die Biomasse enthaltende Vorrichtungsteil der Hochdruckmikrowelle ein Druck zwischen 2 bar bis 30 bar, vorzugsweise 5 bar bis 25 bar, insbesonder 10 - 20 bar erreicht wird.

9. Verfahren nach Anspruch 1 bis 8, **dadurch gekennzeichnet, dass** vor Stufe (b) der Biomasse ein Hydrolyse-Katalysator zugesetzt wird, wobei dieser Katalysator vorzugsweise ausgewählt ist aus einer Mischung aus Redox- und Oxidationsmittel, vorzugsweise einem Mischung ausgewählt aus CuO/Cr₂O₃, ZnO/Cr₂O₃ oder CuO/Zn0.

10. Verfahren nach Anspruch 1, 2, 3, 4 oder 5, **dadurch gekennzeichnet, dass** der Vergärungsprozeß in Stufe (c) und/oder gegebenenfalls der Vorvergärungsprozess in Stufe (d)
• mikrobiologisch, enzymatisch oder sowohl mikrobiologisch als auch enzymatisch; und/oder
• bei einer Temperatur von zwischen 50 und 60 °C, vorzugsweise bei 55°C, oder bei einer Temperatur von zwischen 35 und 45 °C, vorzugsweise bei 40°C; und/oder
• der Vergärungsprozeß in einem Hochleistungsreaktor mit Faulraumbelastungen > 5 kg oTS/m³ Faulraum;
erfolgt.

11. Verfahren nach Anspruch 1, **dadurch gekennzeichnet, dass** die wasserhaltige lignoceellulosehaltige Biomasse ausgewählt ist aus:
Mais, Maissilage, Gras, Grassilage, Ganzpflanzengetreide, Silage aus Getreideganzpflanzen, Stroh, Dung; Garten- oder Forstabfall wie Zweigen und Grünschnitt.

12. Verfahren nach Anspruch 1, 2, 3, 4 oder 5, **dadurch gekennzeichnet, dass** die Biomasse zwischen den Stufen (a) und (b), zwischen den Stufen (d) und (b) und/oder zwischen den Stufen (c) und (b) auf Temperaturen bis zu 150 °C, vorzugsweise 140°C erwärmt wird, vorzugsweise durch Wärmeaustauschgeräte.

13. Verfahren nach Anspruch 1, 2, 3, 4 oder 5, **dadurch gekennzeichnet, dass** die Biomasse zwischen den Stufen (b) und (c) von Temperaturen oberhalb von 150 °C, vorzugsweise oberhalb von 165°C abgekühlt wird auf Temperaturen unterhalb von 60 °C, vorzugsweise unterhalb von 56°C, vorzugsweise durch Wärmeaustauschgeräte.

14. Verfahren zur Herstellung von Biogas oder Bioethanol gemaß Anspruch 1, 2, 3, 4 oder 5, **dadurch gekennzeichnet, dass**
• in einer Stufe (a) die lignocellulosehaltige Biomasse durch ein Zerfaserungsgerät aufbereitet wird;
• in einer nachfolgenden Stufe (b) die optional zwischenbehandelte, aufbereitete lignocellulosehaltige Biomasse aus Stufe (a) zunächst in einer thermischen Hydrolyse (b1) in einer Hochdruckmikrowelle auf Temperaturen zwischen 150 und 250 °C erwärmt und hydrolysiert wird und anschließend in einem Druck-reduzierungsschritt (b2) der in der Hochdruckmikrowelle entstandene Druck in der Biomasse durch Kontaktieren der die Biomasse führenden Vorrichtung mit einem Raum mit niedrigerem Gasdruck reduziert wird;
• in einer nachfolgenden Stufe (c) die optional zwischenbehandelte hydrolysierte lignocellulosehaltige Biomasse aus Stufe (b) einem anaeroben Vergärungsprozess unterworfen wird.

15. Verfahrensvorrichtung zur Herstellung von Biogas oder Bioethanol aus wasser- und lignocellulosehaltiger Biomasse mit mindestens folgenden durch zum Transport von Biomasse geeigneten Röhrensystemen verbundenen Vorrichtungen, wobei diese in Reihe miteinander oder über weitere Vorrichtungen aufeinander folgend verbunden sind:
i) Stoffannahmevorrichtung (S) zur Aufnahme, zum Sammeln und/oder zum Einspeisen der lignocellulosehaltigen Biomasse in die Verfahrensvorrichtung,
ii) Zerfaserungsgerät oder Extruder (Z) zur Zerkleinerung und Zufaserung der lignocellulosehaltigen Biomasse und
iii) Erster Faulbehälter (F1) zur anaeroben Vergärung,
**dadurch gekennzeichnet, dass** zwischen Zerfaserungsgerät/Extruder (Z) und Erstem Faulbehälter (F1) als weitere Vorrichtung eine Mikrowellenanlage (MWA) mit Hochdruckmikrowelle eingefügt ist, die mit Extruder (Z) und Erstem Faulbehälter (F1) durch zum Transport von Biomasse geeignete Röhrensysteme direkt oder über weitere Vorrichtungen verbunden ist.

16. Verfahrensvorrichtung gemäß Anspruch 15, **dadurch** gekenzeichnet,
dass zwischen dem Extruder (Z) und der Mikrowellenamlage (MWA) mindestens ein zweiter Faulbehälter (F2) zur anaeroben Vergärung angeordnet ist, der mit dem Extruder (Z) und der Mikrowellenanlage (MWA) durch zum Transport von Biomasse geeigneten Röhrensysteme direkt oder über weitere Vorrichtungen verbunden ist; und/oder
dass der Erste Faulbehälter (F1) mit der Mikrowellenanlage (MWA) durch weitere zum Transport von Biomasse geeignete Röhrensysteme direkt und/oder über weitere Vorrichtungen so verbunden ist, dass Biomasse vom Ersten Faulbehälter (F1) zur Mikrowellenanlage (MWA) transportiert wird; oder
dass der Erste Faulbehälter (F1) mit der Mikrowellenanlage (MWA) durch weitere zum Transport von Biomasse geeignete Röhrensysteme über den Extruder (Z) und/oder mindestens einen Zweiten Faulbehälter (F2) und gegebenenfalls weitere Vorrichtungen so verbunden ist, dass Biomasse vom Ersten Faulbehälter (F1) zur Mikrowellenanlage (MWA) transportiert wird.
